# EUROPEAN PATENT APPLICATION

(11) **EP 2 926 813 A1**
(43) Date of publication of application: **07.10.2015**
(21) Application number: 13860230.5
(22) Date of filing: 01.11.2013
(51) Int. Cl.: A61K 31/365, A61K 31/155, A61P 3/04

(54) **METFORMIN-ORLISTAT COMPOSITIONS**

(30) Priority: 03.12.2012 MX 2012014070
(71) Applicant: Landsteiner Scientific S.A. de C.V., 01900 Distrito Federal (MX)
(72) Inventor: GARCÍA MONDRAGÓN, Maria Lourdes, Estado de México (MX); GÓMORA PACHECO, Marisol Juana, C.P. 51350 Zinacantepec Estado de México (MX); RODRÍGUEZ BERNAL, Patricia María, C.P. 50160 Toluca Estado de México (MX)
(74) Representative: Covadonga Fernández-Vega Feijoo, Maria
(86) International application number: PCT/MX2013/000133
(87) International publication number: WO 2014/088386

(57) **Abstract**

This invention provides a combination of a hypoglycemic, metformin, and an irreversible intestinal gastric and pancreatic lipase inhibitor, Orlistat, for a better control of obesity than with the monotherapy used with each of them; this composition provides "low" doses of both drugs to minimize secondary effects and to make use of the synergic effect of both compounds.

## Description

### FIELD OF THE INVENTION

This invention is related to composition of a hypoglycemic and a gastric and pancreatic lipase inhibitor in the intestine, particularly combinations of Metformin and Orlistat, useful in the prevention and treatment of obesity and overweight.

### BACKGROUND

Diabetes is a chronic disease which occurs when the pancreas does not produce sufficient insulin, or when the body does not make an effective use of the insulin that it produces. This leads to hyperglycemia; the metabolism of lipids, carbohydrates and altered proteins; and a great risk of vascular complications. Type 2 diabetes mellitus (T2DM), formerly known as non-insulin dependence or adult diabetes is found in 90% of all people in the world who have diabetes, and it is a world health problem. The World Health Organization published in its webpage that 346 million people throughout the world have diabetes, and in 2004 3.4 million died from consequences of the high levels of blood sugar. A recent study made using information from 91 countries reported that 6.4% of all adults had diabetes in 2010, affecting 285 million adults; and that number will increase to 7.7% and 439 million in adults by the year 2030. In Mexico diabetes among adults between 20 and 79 years of age is estimated at 10.1% in 2010 and 13.3% in 2030. This condition also leads to causes of death in that country.

It is interesting that approximately 90% of the cases of T2DM in Mexico can be attributed to obesity and overweight. In fact these two conditions are very closely tied, and favor the development of other chronic diseases such as breast and endometrial cancer, hypertension, brain infarction, among others. It is known from a good source that moderate weight loss of 5-10% is related to a significant reduction in serum sugar, blood pressure and lipid levels, as well as other risk factors associated with this condition.

The risk of death also increases with a body mass index of ≥ 25 Kg/m². For this reason, achieving a healthy weight and preventing a weight gain are integral components of an optimum management of diabetes.

Traditional treatment of T2DM has focused on correcting hyperglycemia; and anti-hyperglycemic therapy is often initiated with metformin. Metformin, a biguanide that reduces the production of hepatic glucose, increases the absorption of glucose and use of skeletal muscle and reduces the absorption of carbohydrates. In addition, other non-glycemic benefits attributed to metformin include an improvement in lipids with a moderate weight loss or no weight gain, as well as an improvement in vascular reactivity and endothelial and micro vascular function. Metformin is absorbed mainly in the small intestine, has an oral bioavailability of 50-60% when fasting. Its blood protein link is insignificant, and as seen by its volume its distribution is very evident.

It is not metabolized by the liver and close to 90% is excreted with no changes in the urine. Orlistat on the other hand irreversibly inhibits gastric and pancreatic lipases, preventing the collapse of dietetic fat to fatty acids and glycerol. Therefore fat absorption decreases and with the respective increase in its excretion with up to 30% of dietetic fat excreted in feces. Together with a low calorie diet in obese individuals it produces a moderate but steady weight loss. In a study performed in patients with type 2 diabetes treated with metformin, orlistat or placebo was added, after 1 year of treatment orlistat increases weight loss and improves glycemic control, lipid levels in blood, and blood pressure in obese patients with type 2 diabetes than those treated with metformin. The metformin-orlistat combination in addition seems to be safe in acute and chronic treatment. Orlistat has a bioavailability below 5% when administered orally, and is detectable in blood only through mass-chromatograph spectrometry of liquids or mass-chromatograph spectrometry of gases with a low detection limit. Practically 97% of orlistat is excreted in feces.

Application US-20100113581, published May 6, 2010 and entitled "Combination therapies for the treatment of obesity" describes pharmaceutical compositions including diethylpropion, metformin, orlistat, and at least one pharmaceutically acceptable carrier, as well as treatment methods for patients who suffer obesity or who need to lose weight. Application US-20100113580 likewise describes compositions that include bupropion, metformin, orlistat, and at least one pharmaceutically acceptable carrier, as well as a treatment method for patients who suffer obesity or who must lose weight. Application US-2007/0060532 of March 15, 2007, "Use of Metformin and Orlistat in the treatment or prevention of obesity" describes combinations of orlistat and metformin in amounts of between 50 mg and 750 mg of orlistat and 100 to 1000 mg of metformin, co-administered to mice either simultaneously or in sequence.

### PURPOSE OF THE INVENTION

The purpose of this invention is to provide a combination of one hypoglycemic, such as metformin, and an irreversible gastric and pancreatic lipase inhibitor in the intestine such as orlistat, to better control obesity than when used in a monotherapy with each.

A second purpose is to provide a pharmaceutical composition that contains both drugs in a single pharmaceutical form, which permits an easy administration and therefore improves the patient's tendency to follow the treatment.

The purpose of the invention is also to allow the use of "low" doses of both drugs to minimize secondary effects, and thereby take advantage of the synergic effect of both compounds.

### BRIEF DESCRIPTION OF THE FIGURES

Table 1. Demographic characteristics of healthy Mexican volunteer subjects. Data is presented as an average ± standard deviation.
Table 2. Pharmacokinetic parameters obtained with tablets of 500 mg of metformin alone and in combination with 60 mg of orlistat after administering a single dose to 26 healthy Mexican volunteers.
   The data is presented as the average ± standard deviation. The variation coefficient in the percentage is shown in parenthesis.
   a standard deviation. The variation coefficient in the percentage is shown in parenthesis. (sic)
Table 3. Relative bioavailability of metformin in combination with orlistat (B) in relation to metformin alone (A). The results are provided as intervals of confidence of 90% for Cmax and the area under the curve (AUC). The table likewise shows the probability of obtaining values outside of fixed limits and potency. The limits were set at between 80 - 125%.

**Table 1**

| Charateristics | Sample Size (n=26) |
|---|---|
| Men | 50.0% |
| Women | 50.0% |
| Age (years) | 29.0 ± 4.9 |
| Weight (Kg) | 66.1 ± 10.5 |
| Waist (cm) | 78.6 ± 8.0 |
| Hips (cm) | 91.5 ± 6.1 |
| Height (cm) | 162.8 ± 10.6 |
| BMI (kg/m²) | 24.8 ± 1.9 |
| Waist-hip ratio | 0.84 ± 0.07 |
| Waist to height ratio | 0.47 ± 0.03 |

**Table 2**

| Parameter | Metformin | Metformin + Orlistat |
|---|---|---|
| Cmax (µg/mL) | 1.39 ± 0.44 (31.4) | 1.38 ± 0.48 (34.9) |
| Tmax (h) | 2.41 ± 1.30 (53.9) | 2.40 ± 1.08 (45.0) |
| AUC₂₄ₕ (µg h/mL) | 7.59 ± 3.17 (41.8) | 7.80 ± 2.83 (36.3) |
| AUC∞ (µg h/mL) | 8.48 ± 4.13 (48.6) | 9.13± 4.29 (47.0) |
| t_{1/2} (h) | 2.88 ± 1.46 (50.8) | 3.52± 2.55 (72.5) |

**TABLE 3**

| Parameter | Ratio (B/A) | Intervals of Confidence (%) | Probability of excess of any limit | | Potency |
|---|---|---|---|---|---|
| Cmax (µg/mL) | 0.9776 | 87.5-109.3 | P<80% 0.0025 | P > 125% 0.0004 | 0.9867 |
| AUC₂₄ₕ (µg h/mL) | 1.0513 | 88.7-124.7 | P<80% 0.0056 | P > 125% 0.0476 | 0.7906 |

Figure 1. Heart rate, blood pressure, breathing rate, and tympanic temperature measured after a single dose of 500 mg of metformin or 500 mg of metformin plus 60 mg of orlistat. The data are presented as average ± standard deviation for 26 healthy volunteers.
Figure 2. Blood time concentration curves for 500 mg of metformin alone or combined with 60 mg of orlistat in 26 healthy volunteers. The data are presented as average ± standard deviation.

### DESCRIPTION OF THE INVENTION

According to the evidence, it is plausible to provide a combination of metformin-orlistat for a better treatment than the individual administration of each drug, as this treatment would improve compliance in obese patients with T2DM.

This invention allows the determination that the co-administration of orlistat has no influence on the pharmacokinetics of metformin; therefore it is possible to assure that orlistat is a useful drug to help the activity of metformin in producing weight loss and improving glycemic control, serum lipid levels and blood pressure in obese patients with type 2 diabetes.

The compositions of this invention include between 200 and 600 mg of metformin and between 40 and 100 mg of Orlistat, and in addition 10 to 15% of the total weight of the composition is of one or more pharmaceutically acceptable excipients. Forms of oral dosage are the compositions preferred for use in this invention and that are known for this administration, for example tablets, coated tablets, capsules, microspheres, and grains. Pharmaceutically acceptable excipients used in preparing the compositions are those known for each form used in the preparation of the compositions are those known for each pharmaceutical form (sic). Capsules and tablets can be prepared from a mixture of the active ingredients with diluent agents such as calcium phosphate, mannitol, microcrystalline cellulose, lactose, DC mannitol, mannitol powder, sorbitol, isomalt, disintegration agents such as cornstarch, crospovidon, croscarmellose sodium, lubricating agents such as magnesium stearate, sodium stearyl fumarate, talc, sodium lauryl sulfate, agglutinants such as polyvinylpyrrolidone, hydroxipropyl methylcellulose (for any substitution), ethyl cellulose, gum Arabic, hydroxypropyl cellulose (for any substitution), gelatin, hydrolyzed collagen, sliding agents such as, colloidal silicon dioxide, talc, stearic acid and similar.

In addition, tablets can include an aesthetic coating of hydroxipropyl methylcellulose phthalate, polyvinyl alcohol, polymethyl crylates, Opadry II, Nutreateric, Acryleze, Sureteric or Eudragit, as well as soft and/or hard gelatin capsules can be prepared from a mixture of the active ingredients and appropriate excipients, or with granules produced in accordance with known techniques and from the compositions described here.

### Example 1. Composition of 500 mg of metformin and 60 mg of Orlistat in capsules.

| Active ingredient | |
|---|---|
| DC metformin chlorhydrate 90 equivalent to chlorhydrate of metformin | 555.55 mg |
| Orlistat | 60 mg |

| Excipients | |
|---|---|
| Hydrolyzed gelatin | 6.0 mg |
| Gum Arabic | 6.0 mg |
| Glycerine | 1.2 mg |
| Mannitol | 36.0 mg |
| Colloidal silica dioxide | 9.6 mg |
| Sodium stearyl fumarate | 1.2 mg |
| Purified water | sq |
| Total | 675.555 MG |

| | |
|---|---|
| sq: sufficient quantity | |

### PROCEDURE

### 1. GRANULATION OF ORLISTAT

Dissolve gelatin, glycerin and gum arabic in water; add one part of colloidal silica dioxide to form a viscous suspension, white in color; pass Orlistat and a part of the colloidal silica dioxide through a sieve, mix and granulate with the previous suspension; dry in fluidized bed at a temperature of 30°C to 35°C, to form coated granules.

Mix with metformin chlorhydrate and mannitol; lubricate with sodium stearyl fumarate and one part of colloidal silica dioxide; finally encapsulate.

### Example 2: Composition of 300 mg of metformin and 40 mg of Orlistat in tablet

| Active ingredient | |
|---|---|
| Metformin chlorhydrate | 300 mg |
| Orlistat | 40 mg |

| Excipients | |
|---|---|
| Hydrolyzed gelatin | 3.8 mg |
| Gum Arabic | 3.8 mg |
| Glycerin | 0.7 mg |
| Mannitol | 23.2 mg |
| Colloidal silica dioxide | 6.2 mg |
| Sodium stearyl fumarate | 0.7 mg |
| Purified water | sq |
| TOTAL | 378.40 mg |

| | |
|---|---|
| sq: sufficient quantity | |

The tablets are prepared as follows:
Dissolve hydrolyzed collagen in water, dissolve glycerin and gum Arabic; add one part of colloidal silica dioxide to form a viscous suspension; sieve Orlistat and one part of colloidal silica dioxide, mix and granulate with the previous suspension, dry in fluidized bed. Mix with metformin chlorhydrate and mannitol, lubricate with sodium stearyl fumarate and one part of colloidal silica dioxide. Finally compress.

### Example 3 Composition of 500 mg of metformin and 60 mg of Orlistat in coated tablets.

Tablets obtained in accordance with Example 2 are coated with a watery solution of Opadry II.

The compositions object of this invention were tested in the clinical study described below, which shows that the compositions object of this invention comply with pharmacological effects for the treatment of obesity and overweight.

### Subjects

Twenty six healthy volunteers, men and women, between 22 and 40 years of age with a body mass index of < 28 Kg/m², waist-height ratio of ≤ 0.5 and waist-hip ratio of <0.8 for women and <0.95 for men. All subjects agreed to participate in the study and were considered as healthy, all as determined through detection tests including medical history, physical examination, electrocardiogram, hematic biometry, blood chemistry with 24 elements, detection of drug abuse, general urine exam as well as hepatitis B and HIV tests. Vital signs were considered normal with blood pressure of 50-90 / 84-120 mmHg, heart rate of 50-100 pulses/min, breathing rate was 14-25 breaths/min, and tympanic temperature of 36-37.8°C. Registry criteria also excluded subjects who had any drug prescribed within a period of 2 weeks prior to entering the study or who had consumed xanthine, alcohol, grilled food, or grapefruit juice 72 hours before or during the experiment. Smokers, pregnant women and subjects with a clinical history of hypersensitivity to drugs, foods or environmental substances were not included.

The experimental protocol was designed in accordance with general ethical principles set forth in the Helsinki Declaration. The protocol for this study as well as the informed consent documents were completely reviewed and approved by the Ethics Committee of Biomagno, S. A. de C.V. with number 02-LTSF-03-MTN/RLT-1 in April, 2011.

### Study Design

The prospective and longitudinal study was performed in a crossed and balanced, randomized design with two sequences, two periods, a single dose, single blind with a wash out period of 1 week between treatments. A single dose of 500 mg of metformin form the commercially available product of reference Dabex® (Tablets, Merck S.A. de C.V.) or 500 mg of metformin plus 60 mg of Orlistat was administered with 250 mL of water after 10 hours of fasting. After dosing, serial blood samples were collected and vital signs monitored during a period of 24 hours. Blood samples of 10 mL each were extracted at 0, 0.25, 0.5, 0.75, 1, 1.25, 1.5, 1.75, 2, 3, 4, 6, 8, 12 and 24 hours after administration. Vital signs were measured at 0, 1, 2, 3, 4, 6, 8, 12 and 24 hours after dosing. Subjects were held for 36 hours and monitored for safety and adverse effects throughout the study. Volunteers received a diet calculated at 3000 cal/day in each period.

### Analytical Method

Preparation of the sample involved separation with acetonitrile and dichloromethane. 0.5 mL of blood was added to 1.5 mL of acetonitrile. The mixture was mixed at maximum speed for 30 seconds and centrifuged at 2,000 rpm for 10 minutes. The supernatant was mixed with 1.5 mL of dichloromethane and the new mixture was mixed again and centrifuged under the conditions mentioned in the previous step. Afterwards 50 µL of supernatant was injected into the chromatograph system. A calibration curve includes the following concentration points: 0.05, 0.1, 0.2, 0.5, 1 and 1.5 µg/mL. Control samples were prepared using human blood with a biological matrix and appropriate amounts of drug to obtain concentrations of 0.15, 0.4 and 1.2 µg/mL.

A high performance liquid chromatograph coupled to an ultraviolet detector (HPLC/UV) was used to determine serum concentrations of metformin. The chromatograph system was comprised of a high resolution liquids chromatograph (Waters corporation) comprised of a model 515 pump, a model 717 auto-sampler tray, a UV detector model 2487 and Empower 2.0 software for data analysis. Separation was completed in a Resolve silica column 150 mm long, 3.9 mm internal diameter and 90A particle size, using a mobile phase of monobasic sodium acetonitrile phosphate 0.03 M (25:75 v/v) with a flow rate of 1.2 mL/min. Temperature of the self-sampler tray was 4°C and metformin retention time was 3.5 - 4.5. Absorbency was read at 234 nm.

This method was developed and validated for specificity, sensitivity, linear recuperation, precision, accuracy, stability and robustness in our laboratory according to the specifications of NOM-177-SSA1-1998. Quantification of the metformin had no interference from acetaminophen, acetylsalicylic acid, salicylic acid, ibuprofen or heparin. In addition the method was lineal in a range of 0.05 - 1.5 µg/mL ) (r = 0.998), its quantification limit was 0.025 µg/mL with 83.22% absolute recovery of metformin. Control sample accuracy was 0.15, 0.4 and 1.2 µg/mL concentrations was 8%, 3% and 1.75%, respectively. The intra assessment variation coefficient was 4.15%, 3.28% and 2.40% respectively; and the inter assessment variation coefficient was 3.46%, 10.25% and 6.75% respectively. No significant degradation was observed in the metformin during the cycles of freezing/thawing, short and long term storage or processing conditions.

### Pharmacokinetic and Statistical Analysis

The sample size was calculated based on a crossed design with data transformed by logarithm, considering an intra individual variation coefficient of 20%, power of 80% and significancy level of 5% according to Chow and Wang. Under these conditions the calculated sample size was 18 volunteers; as such 26 volunteers were registered, taking into consideration withdrawals and potential abandonments.

Concentration curves for serum metformin over time were constructed for each volunteer and for each formulation. The highest observed serum concentration and corresponding time was defined with the values Cₘₐₓ and Tₘₐₓ, respectively. The elimination rate constant (Kₑ) was obtained through a linear regression of the best adjusted slope of the terminal logarithmic linear decrease in serums concentrations against the time profile. Half life (t_{1/2}) was obtained as 0.693/Kₑ. The area under the serum concentration curve at the last quantifiable concentration (Cₜ) in time t (AUC_{0 - t}) was determined through a linear trapezoidal integration. AUC extrapolated to infinite (AUC_{0 - ∞}) was calculated as AUC_{0 - t} = Cₜ/Kₑ. Pharmacokinetic parameters were generated using the Professional WinNonlin software version 2.0 (Pharsight, Palo Alto, CA, USA).

In order to not establish any pharmacokinetic interaction bioequivalence was assessed using a variance analysis (ANOVA) for a crossed study design considering sequence, period and treatment effects. The AUC and Cₘₐₓ values for each volunteer were logarithmically transformed and ratios between both formulations calculated [logarithm (AUC₂₄ₕ^{B}/AUC₂₄ₕ ^{A}) and logarithm (Cₘₐₓ^{B}/CₘₐₓA)]. The mean was then obtained and 90% Cl of the ratios of AUC₂₄ₕ and Cₘₐₓ. Bioequivalence between metformin alone and metformin combined with Orlistat was determined when 90% of Cl of these two parameters were between 0.8 and 1.25 and when p ≤ 0.05 after a Schuirmann unilateral hypothesis test.

### Results

After two treatment periods 7 adverse effects had occurred, 3 of which were observed with metformin alone and 4 with metformin plus Orlistat. The system most affected was the digestive tract with 3 abdominal colics and 3 abdominal distensions; and the nervous system with one headache. Women were more likely to suffer adverse effects (5 of 7) due to the treatment. All volunteers completed the study as the adverse effects were light.

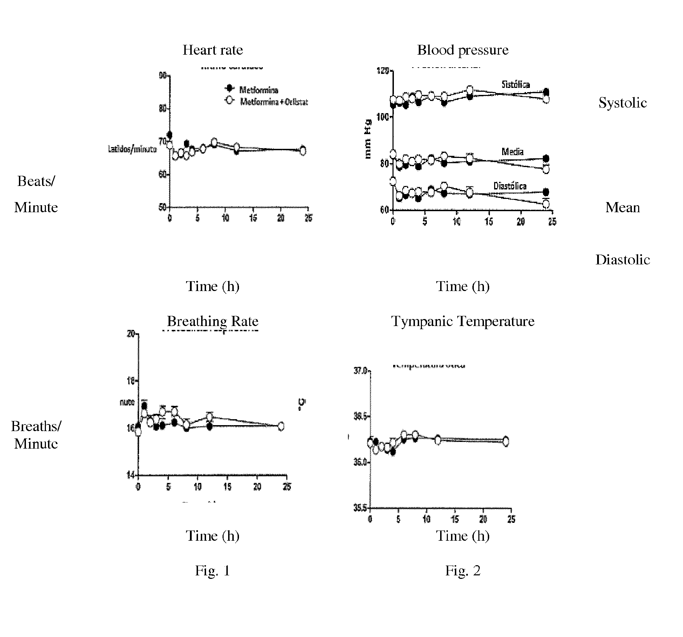

The anthropometric characteristics of 26 Mexican volunteers finally included in this study were ages between 21 and 40 years, 50% of whom were women. Body mass index varied from 20.5 to 27.67 Kg/m². The mean ± standard deviation of these and other parameters are set out in Table 1. The values obtained in this respect by electrocardiogram, hematic biometry, blood chemistry, detection of drug abuse, general urine test and hepatitis and HIV tests showed normal values for all volunteers (data not shown); thus verifying their healthy condition. During the study the volunteers's vital signs were monitored. Figure 1 shows heart rate (50-100 pulses/min), blood pressure (50-90 / 84-129 mmHg), breathing rate (14-25 breaths/min), and tympanic temperature (36-37.8° C) were within normal values throughout the study. Likewise there was no statistical difference in vital signs due to the treatment, as seen through repeated ANOVA bilateral measurements.

Plasma-time concentration curves for both formulations show that the concentration of metformin in blood decreased multi-exponentially after the peak concentration time (Figure 2). The mean ± standard deviation for metformin tablets and metformin plus Orlistat capsules were 1.39 ± 0.44 and 1.38 ± 0.48 µg h/mL for Cₘₐₓ; and 7.59 ± 3.17 and 7.80 ± 2.83 µg h/mL for AUC₂₄ₕ, respectively. These and other pharmacokinetic parameters not considered in determining bioequivalence are shown in Table 2. The bioequivalence analysis for two Schuirmann unilateral hypothesis tests on both formulations of metformin showed that 90% of Cl for Cₘₐₓ and AUC₂₄ₕ were between 80% and 125%, with a probability of excess for any limit less than 0.05 (Table 3). In addition, the ANOVA analysis showed that there was no sign of variability for the sequence, formulation period in the pharmacokinetic parameters.

### Discussion

The blood-time concentration curves for metformin alone and combined with Orlistat were almost perfectly transposed, indicating that Orlistat did not modify the pharmacokinetic parameters of metformin when both drugs are administered together.

The AUC of metformin-orlistat was slightly superior to metformin alone. The pharmacokinetic results obtained in the current study shows conclusive data with regards to therapeutic equivalence, as the comparison between the test formulation, metformin-orlistat, and the reference formulation, metformin, for Cₘₐₓ and AUC₂₄ₕ showed percentages that fell within the scale of equivalence.

## Claims

1. Pharmaceutical compositions to prevent and treat obesity are so characterized because they contain between 200 and 600 mg of metformin and between 40 and 100 mg of Orlistat; and from 10 to 15% of the total weight of the composition is one or more pharmaceutically acceptable excipients.

2. The compositions according to claim 1, which contains 500 mg of metformin and 60 mg of Orlistat.

3. The compositions according to claim 1, which contains 300 mg of metformin and 40 mg of Orlistat.

4. The compositions according to claim 1, which has an excipient that includes hydrolyzed collagen; gum Arabic; glycerin; mannitol; colloidal silica dioxide; sodium stearyl fumarate.

5. The compositions according to claim 1, which has forms of oral dosage which are tablets, tablets with enteric coating, pills, capsules, microspheres and granules.

6. The compositions according to claim 1, where tablets can also include an enteric coating of hydroxypropyl-methylcellulose-phthalate, Opadry II, Nutrateric, Acryleze, Sureteric, Eudragit.

7. The composition according to claim 1, where the dose resists the gastric phase.

8. A production process for the composition according to the claim, which consists of dissolving in hot water at 40° C gelatin, glycerin and Arabic gum, adding one part of colloidal silica dioxide, forming a viscous, white color suspension, sieving Orlistat and one part of colloidal silica dioxide, mixing and granulating with the previous suspension, drying in fluidized bed to form coated granules. Mixing with metformin chlorhydrate and mannitol, lubricating with sodium stearyl fumarate and one part of colloidal silica dioxide.

9. The process according to claim 8, which also includes the stage of encapsulating the mixture obtained.

10. The process according to claim 8, which also includes the compression stage to obtain a tablet, which can also be coated with an enteric coating.
